# EUROPEAN PATENT SPECIFICATION

(11) **EP 3 720 531 B1**
(45) Date of publication and mention of the grant of the patent: **27.12.2023**
(21) Application number: 18886367.4
(22) Date of filing: 04.12.2018
(51) Int. Cl.: A61M 16/00, A61H 23/00

(54) **A SYSTEM FOR RESPIRATORY SECRETION MANAGEMENT**
SYSTEM ZUR VERWALTUNG DER ATEMWEGSSEKRETION
SYSTÈME DE GESTION DES SÉCRÉTIONS RESPIRATOIRES

(30) Priority: 05.12.2017 SG 10201710079P
(43) Date of publication of application: 14.10.2020
(73) Proprietor: Agency for Science, Technology and Research, Singapore 138632 (SG)
(72) Inventor: CHONG, Mark Seow Khoon, Singapore 139955 (SG); PRASAD, Kenath Priyanka, Singapore 139955 (SG); TAN, Ee Lim, Singaproe 139955 (SG); MATHEWS, Ian, Singapore 139955 (SG)
(74) Representative: HGF
(86) International application number: PCT/SG2018/050591
(87) International publication number: WO 2019/112517

(56) References cited:
- WO-A1-2016/145483
- CN-A- 104 689 442
- US-A1- 2010 022 923
- US-A1- 2014 190 481
- US-A1- 2014 316 310
- US-A1- 2016 121 062
- US-A1- 2017 291 007
- US-B1- 8 424 527
- US-B2- 8 082 920

## Description

### TECHNICAL FIELD

The present disclosure relates to systems for respiratory secretion management. In particular, the present disclosure relates to systems for applying waves, such as pressure waves, to the respiratory system of a subject for loosening and/or dislodgement of secretion from the airway.

### BACKGROUND

Mucociliary clearance describes the self-clearing mechanism of the respiratory system. It involves the mucociliary escalator which serves to mobilise secretions. The mucociliary escalator thus prevents airway obstructions and maintains optimal function of the respiratory system.

In healthy individuals, 10-100 ml of airway secretions are continuously produced and cleared by the mucociliary escalator.

This mucous clearance mechanism may be compromised by a variety of factors, including pathophysiological conditions affecting the mucociliary escalator - such as bronchiectasis and ciliary dyskinesia - and disorders that alter the production and composition of mucus - such as sinusitis, cystic fibrosis and chronic obstructive pulmonary disease (COPD). The resulting stasis of secretions obstructs conducting airways, providing a nidus for recurrent infections and inflammatory responses, leading to repeated insults to the airways and parenchyma.

For these reasons, regular use of airway clearance techniques (ACTs) and devices are critical for effective mucus mobilization and expectoration for those with negatively affected mucociliary escalator. This helps to prevent recurrent infections thereby preserving long-term lung function.

ACTs typically employ physical and mechanical means to manipulate airflow in the respiratory system. The intention is to mobilise secretions from distal sections of the respiratory system towards easily evacuated parts of the respiratory system such as, from the distal airways towards the central airways, wherein evacuation is effected by coughing or natural swallowing. Physical manipulations include breathing manoeuvres, postural drainage, and manual techniques. Mechanical devices, including chest percussion devices and oscillatory pressure devices function on principles of altering air flow, leading to and/or generating an evacuation-like effect.

Regardless of the intervention technique, ACTs are generally time-consuming, and thus likely responsible for poor adherence to the prescribed therapy. The use of devices and prescribed therapies can require two to six hours of active patient involvement every day and has been described as a major factor contributing to reduced compliance rates to ACT in cystic fibrosis (CF) patients.

Stasis of secretions in respiratory diseases leads to chronic infection, inflammation and lung destruction. Respiratory physiotherapy has been used for many years to help in removal of secretions. However, the lack of availability and accessibility to adequate therapy outside of a clinical setting have led to the advent of mucus clearance device development.

Early devices were aimed at using robotic means to either self-administer percussive therapy or to manipulate airflow in order to effect mucus clearance. The latter includes positive expiratory pressure devices developed in the 1970's and first introduced in the United States as an alternative to conventional physiotherapy. The mechanism of action lies in (i) splinting airways open to allow movement of secretions and (ii) allowing air behind secretions, pushing them towards larger airways during forced expiration.

High Frequency Chest Wall Oscillations (HFCWO) were then found to be able to loosen mucus from airways. HFCWO elicit fluctuations in air-flow during respiration, resulting in "mini-coughs". Originally embodied as respirators with oscillating airflows, the devices have since evolved to deliver oscillating pressures externally via a pneumatic vest which surrounds the thorax. These air pulses compress and release the chest repeatedly and rapidly, leading to vibrations that cause transient flow increases in the airways, loosening mucus and producing cough like shear forces. The first HFCWO vest was licensed in 1988, representing a move towards "passive systems" which were not dependent on the effort of the patient.

This was succeeded by a trend in the 1990's to develop miniaturised, patient-powered devices combining features of oscillation with positive expiratory pressure (PEP). Termed Oscillatory Positive Expiratory Pressure (OPEP), these devices typically comprise vibration systems which produce positive expiratory pressure and cyclic oscillation of the airways during expiration.

Taken together, these trends suggest a dearth of devices which combine features of the above, highlighting the as-yet unmet need for miniaturised and discreet devices that may be used by patients in an unobtrusive manner. US 2010/22923 is directed to an active respiratory therapeutic device for clearing breathing passages, loosening and breaking up mucus plugs and phlegm in a patient's sinuses, trachea, bronchial passages and lungs while a patient is breathing normally through the device is disclosed. The apparatus preferably includes a C shaped curved hollow housing having a closed end portion and an open threaded end portion. The open end portion forms at least part of an acoustic coupling chamber. A generally funnel shaped tapered mouthpiece tapers to a small end portion sized to be inserted into a patient's mouth. The mouthpiece forms another part of the acoustic coupling chamber. An acoustic signal generator housed within the hollow housing generates and directs acoustic vibrations into and through the coupling chamber. The mouthpiece preferably includes a valve permitting a patient breathing through the mouthpiece to inhale through a valve opening and exhale through a bypass passage around the valve while at the same time coupling the acoustic coupling chamber into the patient's airways.

It is desirable therefore to provide a system for airway secretion management that overcomes or ameliorates one or more of the abovementioned problems in the prior art, or at least provides a useful alternative.

### SUMMARY OF THE PRESENT DISCLOSURE

The invention is defined in the appended independent claim 1. Preferred embodiments are matter of the dependent claims.

Disclosed herein is a system for respiratory system secretion management, comprising:
an oscillatory wave generator for generating a wave for loosening secretion from an airway of a subject;
an module in communication with the oscillatory wave generator, for delivering the wave through a nasal passage of the subject;
a power module configured to power the system; and
a communication module coupled to at least one of the nasal module and the oscillatory wave generator, for selectively communicating power and/or one or more waveform characteristics to the respective nasal module and/or oscillatory wave generator.

In the examples given herein, the term "intranasal module" may be used. However, except where context dictates otherwise, it will be understood that the term "nasal module" or "extranasal module" may be used in its place.

As used herein, the phrase "delivering a wave through a nasal passage" includes embodiments in which the nasal module is positioned in the nasal passage to deliver the wave through the nasal passage - i.e. using an intranasal module. The phrase "delivering a wave through a nasal passage" also includes embodiments in which the nasal module is positioned outside the nasal passage - e.g. on the nose - to deliver the wave into, and thereby through, the nasal passage - i.e. using as extranasal module.

The system for respiratory system secretion management may be a system for airway secretion management.

The nasal module may thus be an intranasal module. In some embodiments, the nasal module may be an extranasal module. According to the invention, the nasal module is an intranasal module.

The oscillatory wave generator may comprise a waveform generator for generating an electrical signal corresponding to the wave, and at least one of:
an amplifier for increasing an amplitude and/or power of the electrical signal; and
an acoustic generator for converting the electrical signal into an acoustic signal, said acoustic signal being the wave.

The oscillatory wave generator may be adjustable to control output waveform characteristics. The waveform characteristics may comprise one or more of frequency, amplitude, intensity, pressure, duration of use - i.e. duration over which the waveform will be applied to the subject - and shape - e.g. sinusoidal, square etc.

The oscillatory wave generator may be located extra-nasally - e.g. where the nasal module is an extranasal module, in that extranasal module, or otherwise separate from the nasal module. Alternatively, the oscillatory wave generator may be located intranasally - e.g. where the nasal module is an intranasal module, in that intranasal module.

The nasal module may be shaped to be positioned in the nasal passage of the subject.

The nasal module may be in communication with the oscillatory wave generator via a waveguide.

The nasal module may comprise a housing shaped to be compatible with - e.g. received in or on, and potentially to grip - the nasal passage of the subject. The nasal module may comprise an acoustic window at a distal end of the housing, through which the wave is delivered into the nasal passage.

The nasal module may be adapted to grip the nasal passage.

The nasal module may comprise an anchor component for preventing irretrievable slippage of the intranasal module into the nasal passage of the subject. The anchor component may comprise a hook for catching onto a columella of the subject.

The communication module may be operable to configure the wave by adjusting the oscillatory wave generator.

The system may further comprise an interface module for providing instructions to the communication module, thereby to control the communication module. The interface module may be configured to instruct the communication module to adjust waveform characteristics of the wave. The interface module may be configured to instruct the communication module to toggle power to the system. The interface module may be configured to instruct the communication module to adjust waveform intensity of the wave.

The interface module and communication module may be in wireless communication. Alternatively, wired communication may be used.

The system may comprise a patch for attachment to facial skin of the subject, the patch comprising at least one of the oscillatory wave generator, power module and communication module. The system may also comprise the nasal module where the nasal module is an extranasal module.

### BRIEF DESCRIPTION OF THE DRAWINGS

Some embodiments of systems for respiratory system secretion management, components of such systems and experimental usages, in accordance with present teachings will now be described, by way of non-limiting example only, with reference to the accompanying drawings in which:
FIG. 1 is a schematic illustration of a system for respiratory system secretion management in accordance with present teachings;
FIG. 2 is an image of the system of FIG. 1 as a physical system for use by a subject (i.e. patient), according to an embodiment of the invention;
FIG. 3 is an image of the system of FIG. 2 in place on a subject;
FIG. 4 is an image of an intranasal module comprising an anchor component;

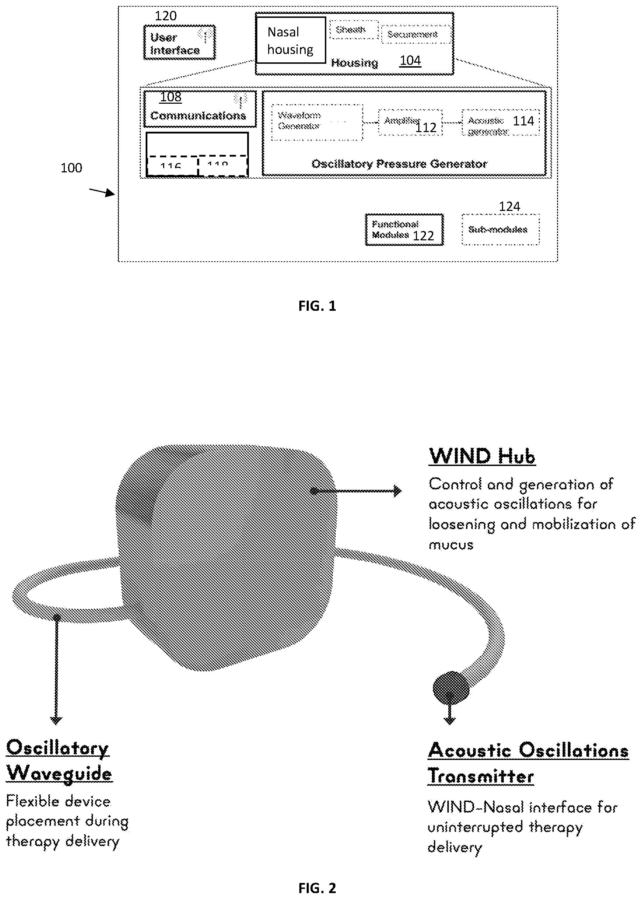

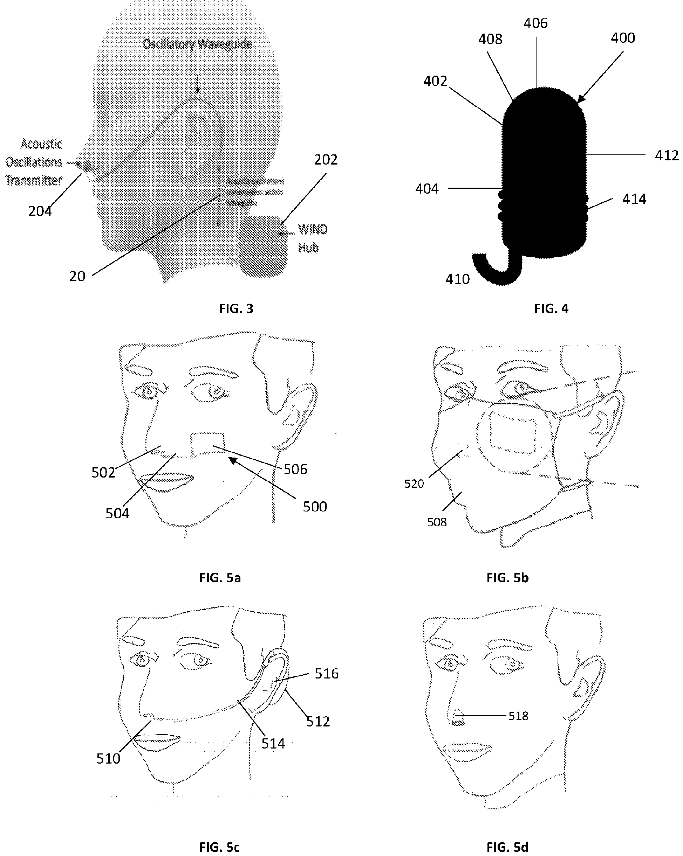

FIGs. 5a to 5d are illustrations of embodiments of the system of FIG. 1 in which: one or more modules of the system are in or on a facial patch connected to an intranasal module - FIG. 5a; one or more modules of the system are in or on a face mask connected to a nasal module - FIG. 5b; one or more modules of the system are in an external housing secured to an ear of the subject, and connected to an intranasal module - FIG. 5c; one or more modules of the system are in an external housing secured to the subject in a hands-free manner during device operation, the eternal housing being connected to an intranasal module; and the intranasal module is located in the nasal passage of the subject;
FIG. 6 is an experimental setup of the system of FIG. 1;
FIG. 7 is an illustration of a subject with an intranasal module located in the nasal passage;
FIG. 8 is a schematic illustration of the system of FIG. 6, located in the nasal passage of a human cadaver for experimentation purposes, and an acoustic meter for determining signal attenuation between a point of origin and a location in the trachea;
FIG. 9 is a pair of images of an experimental setup using a porcine cadaver for determining signal attenuation in the nasopharyngeal region of the porcine cadaver;
FIG. 10 is an image of a bench model used to model signal attenuation;
FIG. 11 shows experimental results taken by measuring an acoustic signal at the source and trachea - in the case of the bench model of FIG. 10, the "trachea" is a location along the bench model that is approximately the same distance as that between source and origin in an adult human; and
FIG. 12 acoustic spectra taken using a digital stethoscope in the region of the anterior apex of the right lung of a subject.

### DETAILED DESCRIPTION

Embodiments of systems for respiratory system, or airway, secretion management as disclosed herein may be used to introduce waves, such as acoustic waves, into the nasal passage of a subject. By being adapted to introduce waves through the nasal passage, wave attenuation between the source and lungs has been shown to reduce. The system thus has greater efficacy in transmission of waves to the lungs and thus, it is considered, shall have greater efficacy in loosening and/or dislodging secretion (e.g. mucus) from the lungs and airways.

The present disclosure provides an intranasal system (which may be a device) which generates oscillatory pressure. This oscillatory pressure, in the form of pressure waveforms, is directed via the nasal passages to lungs, where it serves to loosen and/or dislodge the mucus. As such, the system will be used as an aid in clearing and/or preventing accumulations of mucus in the airways, including the nasal passages, sinus cavities and the bronchial tree.

FIG. 1 shows a system 100 for respiratory system, or airway, secretion management. The system 100 broadly comprises:
an oscillatory wave generator 102;
a nasal module 104;
a power module 106; and
a communication module 108.

While the terms "airway" and "intranasal module" are used in the following discussion of embodiments for illustration purposes, it will be appreciated that those terms may respectively be substituted for "respiratory system" and "nasal module" or "extranasal module" unless context dictates otherwise. The oscillatory wave generator (OWG), hereinafter referred to as OWG module 102, is for generating a wave for loosening secretion from an airway of a subject (not shown). The OWG module 102 is a module capable of generating a wave being, for example, a pressure wave, where that wave may be regular, periodic, aperiodic, variable frequency/power/intensity or any other kind of wave designed to loosen (which may include loosening *per se* and also dislodgement) secretion from the airway of a subject. The OWG module 102 generates the desired waveforms necessary for, for example, mucus clearance.

In the embodiment shown, the OWG module 102, comprises:
a waveform generator 110 (part of an oscillatory waveform generator);
an amplifier 112; and
transducer submodules 114.

The OWG module 102 receives power from the power module 106. The power renders the OWG module 102 operable. The OWG module 102 may be assembled as a single entity or component. Alternatively, the OWG module 102 may comprise multiple components housed separately. For example, the submodules 110, 112, 114 may be housed separately or in combination.

In the present embodiment, the OWG module 102 is adjustable to control output waveform characteristics. The waveform characteristics may comprise, for example, one or both of frequency and amplitude.

Control of the waveform characteristics may be applied at one or more stages in the OWG module 102. For example, the waveform characteristics may be evident in the wave generated by the waveform generator 110. The waveform characteristics may instead be those of the wave as amplified by the amplifier 112 - e.g. the OWG module 102 may define the magnitude of amplification applied by the amplifier 112, to achieve a desired amplitude. Alternatively, the waveform generator 110 may produce a wave of a particular, controlled or predetermined frequency, and the amplifier 112 may then set the amplitude of the wave.

The OWG module 102 may generate a wave having predetermined frequency of between 5 to 5,000 Hz, between a range of 10 - 2000 Hz, around 300 Hz, 500 Hz, 1,000 Hz or 1,500 Hz. The frequency may also vary between consecutive peaks of the wave.

The OWG module 102 may also produce a wave with an expected output sound pressure level (SPL) of 90 dB or higher. The output may be, for example, 100 dB, 105 dB, 110 dB, 115 dB or any other sound level.

The OWG module 102 achieves production of waves conforming within the ranges of 3 to 20 voltage peak-to-peak, which is the voltage input to the system at any point of operation.

In the embodiment, exemplifying the invention, shown in figure three, all three submodules 110, 112, 114 are provided. However, the OWG module 102 may be configured to have the waveform generator 102, and only one of:
the amplifier 112; and
acoustic generator 114.

As discussed with reference to FIGs. 5a to 5d, the OWG module 102 may be housed in the intranasal module, extra-nasally or possibly integrated into a patient-carried mobile device. In some embodiments, submodules 110, 112, 114 may be housed separately. For example, the acoustic generator 114 may be housed in the intranasal module 104, and the waveform generator 110 and amplifier 112 may be housed in an extra-nasal patch or external device. Alternatively, the amplifier 112 and acoustic generator 114 may be in the intranasal module 104, or all three submodules 110, 112, 114 may be in the intranasal module 104.

The waveform generator (submodule) 110 is for generating a signal, e.g. an electrical signal, corresponding to the wave outputted from the OWG module 102. The waveform generator 110 of the present embodiment comprises a tuner responsible for emitting an electrical waveform. The tuner can be adjusted under instruction from the communication module 108, to adjust one or more of the waveform characteristics of the wave.

The waveform generator 110 is currently conceptualised as a stand alone entity. The power received from the power module 106 may be from 3 to 20 voltage peak-to-peak, at any point of operation, and may be, for example, at 3-6V input voltage. The waveform generator 110 and may be accessed or controlled from the communication module 108, via a user interface module (described below), to control output waveform characteristics of frequency and/or amplitude. For example, an output sine wave may be generated at a predetermined frequency, or a predetermined variable frequency, of 5 to 5000Hz, and may be 10 - 2000 Hz, frequencies with up to 120 dB.

The amplifier (submodule) 112 increases the power and/or amplitude of the electrical signal, and may comprise a pre-speaker/transducer module. The pre-speaker/transducer can be used to boost the power or amplitude of output signals. The gain applied to the wave generated by the waveform generator 110 may be any desired gain, such as 50, 60 70, 80 90, up to 100 or over.

The amplifier 112 is used to modulate power of the output, to increase the amplitudes of the generated wave - i.e. pressure wave. Additionally, the amplifier 112 may be used as a means to control the amplitude, in order to cater to user-assessed requirement for efficacy and comfort. In the present embodiment, the amplifier 112 is accessed via a user interface module connected to the communication module 108.

The amplifier 112 may be designed as a stand-alone on-board component or introduced as a DC bias.

The acoustic generator (submodule) 114 converts the electrical signal produced by the waveform generator 110 into an acoustic signal. Where the acoustic generator 114 is provided in the OWG module 102, the acoustic signal outputted by the acoustic generator 114 is the wave outputted by the OWG module 102. The acoustic generator 114 receives the electrical waveform for the waveform generator 110 and converts it into a pressure waveform. In the embodiment shown, the acoustic generator 114 is an electromagnetically-driven speaker - e.g. a miniature speaker. The miniature speaker is functional at a frequency range of 5 - 5000 Hz.

The acoustic generator 114 may be housed within the intranasal module 104. Thus, in use, the acoustic generator 114 may be designed or shaped to fit into the nasal cavity when within the intranasal module 104. Thus, the acoustic generator 114 may be housed within a 10mm diameter x 15 mm long cylindrical space in the intranasal module 104. Alternatively, the acoustic generator 114 may be used with a wave guide or acoustic channel /tunnel to carry the acoustic waves into the nasal cavity. Thus the intranasal module 104 may be in communication with the oscillatory wave generator 102 via the waveguide. In some embodiments, where a signal other than a wave is propagated to the intranasal module 104, the wave guide may be replaced by a wire or other mechanism for communication of signals.

For example, with reference to the system 200 shown in FIG. 2, exemplifying the invention, one or more submodules 110, 112, 114 of the OWG module 102 may be housed in an external housing 202 that is located in the subject's pocket, bag or, depending on shape and size, behind the ear of the subject. As shown in FIG. 3, a wave guide or oscillation waveguide 206 (which may transmit acoustic oscillations) may connect the external housing 202 to the intranasal module 204 (which may include an acoustic oscillations transmitter) over the ear or in any other desired arrangement. The wave guide 206 is flexible so as to permit flexible placement of the external housing during delivery of therapy.

The acoustic generator 114 may be housed within intranasal module 204, the waveform generator 110 or amplifier 112 communicating with the acoustic generator 114 via wave guide 206. Alternatively, the acoustic generator 114 may be housed with the waveform generator 110 or amplifier 112 in the external housing 202, and communicate with the intranasal module 204 via the wave guide 206. Thus, depending on the arrangement of submodules of the OWG module 102, the wave guide 206 may be configured to propagate an electrical wave or signal or an acoustic wave or signal to the intranasal module 104, which then delivers a wave corresponding to the acoustic wave or electrical signal into the nasal passage of the subject.

The intranasal module 104 is in communication with the oscillatory wave generator 102, to deliver the wave generated by the oscillatory wave generator 102 through (e.g. into, so as to progress down) a nasal passage (not shown) of the subject. With reference to FIG. 4, the intranasal module 400 may be shaped to be positioned in the nasal passage of the subject. In the present instance, the intranasal module 400 is bullet shaped.

The intranasal module 104 comprises a housing 402 housing shaped to be compatible with the nasal passage of the subject. The housing 402 contains the in-nose components, including the acoustic components - e.g. acoustic generator 114 - of embodiments described above. Positioning the acoustic generator 114 in the intranasal module 104, rather than in an external housing, reduces the distance the wave - i.e. an acoustic or pressure wave - travels between the source, being the acoustic generator 114, and destination in the subject's airway. There is therefore a reduction in attenuation of the acoustic signal when compared with locating the acoustic generator in an external housing. The same may apply where the waveform generator 110 directly generates the wave that is outputted from the intranasal module 104, and is positioned in the intranasal module 104 rather than in an external housing.

The housing 402 also seals against the nasal passage and thus reduces leakage of acoustic signals. The housing 402 of the present embodiment comprises a bullet-shaped cylindrical casing - presently 10mm diameter x 15mm long, with a 2 mm wall thickness - and a 5mm x 8mm acoustic window 406 on the wall of the distal end 408. The distal end 408 is presently in the shape of a dome. The window 406 allows transmission of the wave, hereinafter referred to as a pressure wave, being a wave produced by the acoustic generator 114. Thus, the wave is delivered through the window 406 into the nasal passage.

The intranasal module 104 may be fabricated by any appropriate method, including blow-moulding, extrusion and/or casting. The intranasal module 104 or the cylindrical portion thereof may be formed from any appropriate material, such as a polymer - for example acrylonitrile butadiene styrene (ABS), polyurethane, polycarbonate or ultra-high molecular weight polyethylene (UHWMPE). These materials are intended to avoid or reduce leakage of the wave. The acoustic window 406 may be fabricated from a sound-conductive material, such as steel.

The intranasal module 104 may be adapted to grip the nasal passage. In other words, the intranasal module 104 may grip a wall of the nasal passage. As shown with reference to FIG. 5b, the nasal module may instead be an extranasal module adapted for positioning outside of the nose, on the nose.

The present intranasal module 400 comprises an anchor component 410. The anchor component 410 prevents irretrievable slippage of the intranasal module 400 into the nasal cavity of the subject. The present anchor component comprises a hook for catching onto a columella of the subject. In some other embodiments, the anchor component may be a circumferential flange that is marginally larger than the nostril of the subject, so as not to fit into the nostril of the subject. In these cases, the anchor component defines the maximum distance at which the distal end 408 (and acoustic window 406) project into the nose of the subject.

The intranasal module 400 further comprises a sheath 412. The sheath 412 provides a waterproof barrier for the intranasal housing 402 and enclosed components. The sheath 412 of the present embodiment is a disposable slip-on, silicon-based polymer sheath, designed to fit over the intranasal housing 402. The sheath 412 may remain attached to the housing 402 by friction fit, and thus be removable by overcoming the frictional attachment. The sheath 412 may alternatively wrap around the housing 402 and connect, for example, to the anchor component 410 or wave guide 206.

The present sheath 412 is 0.2 mm thick. The sheath 412 has embossing - e.g. ribbed structures such as circumferential ribs 414 - to improve grip to the nasal cavity or passage. The sheath 412 may be fabricated by blow-moulding, extrusion, casting and/or calendaring. The sheath 412 may be formed from any appropriate material such as a polyurethane, polyethylene (PE) and polyethylene terephthalate (PET).

The intranasal module 104 may thus be held in place in the nasal passage through a combination of features - i.e. the embossed features described for the sheath 412, and anchor component 410.

The power module 106 is configured to power the system 100. the power module 106 comprises a power source 116 for supply, and may also include a switch 118 for toggling, power to the communication module 108, the wave generator 102 and, where the intranasal module 104 contains some powered components (e.g. the acoustic generator 114 of the wave generator 102), the intranasal module 102. The power source 116 may provide power for all other components for at least one hour of continuous operation.

The form factor of the power module 106 is selected to meet user acceptance for non-obtrusiveness. In the present embodiment, a ⅓ AAA battery with a capacity of 150 mAh may be used, to gain at least one hour of continuous operation. Alternative power sources meeting a specified form factor and power requirements may also be used.

The switch 118 may also be provided in the power module 106, coupled to the power source. The switch 118 is for controlling or toggling operation of the device. These include physical on-device switches, as well as potentially wireless activation or integration into the tuner component described below.

The communication module 108 is coupled to at least one of, and presently both, the intranasal module 104 and the oscillatory wave generator 102, for selectively communicating power and/or one or more waveform characteristics to the respective intranasal module 104 and/or oscillatory wave generator 102. The waveform characteristics may be one or more of power, intensity, amplitude, pressure, frequency, duration of use and shape - e.g. sinusoidal, square etc. The communication module 108 is coupled in the sense that relevant components - e.g. modules 102, 104 and 106 - can be controlled by the communication module 108. The communication module 108 may be wirelessly connected to one or more of those components, or may be connected by hardwire to one or more of those components.

The communication module 108 serves to provide a means for the user to access the device. The communication module 108 is used primarily to (i) toggle power (ii) configure pressure waveforms - e.g. adjust or set amplitude and/or frequency.

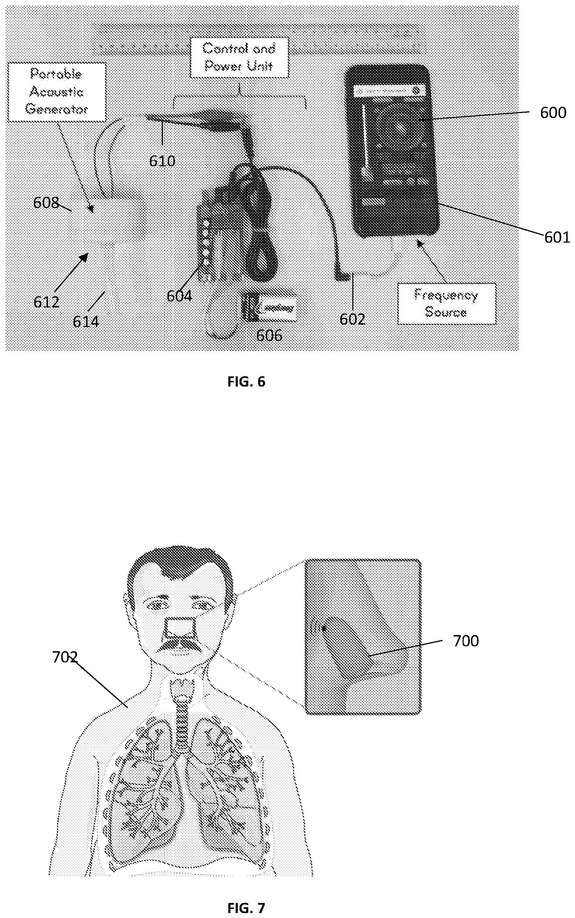

The communication module 108 may be accessed via a user interface 120 for providing instructions to the communication module 108, thereby to control the communication module 108 - i.e. cause the communication module 108 to issue instructions to the wave generator 102 and other components, if necessary. The user interface 120 will provide the user with means to provide instructions, as well as to obtain feedback from the device. The current conception employs a graphical user interface on a mobile device (e.g. user interface 600 shown in FIG. 6) to adjust waveform characteristics, as well as physical or virtual knobs or buttons to toggle power, as well as adjust waveform intensity. As such, a combination of physical and mobile device-based controls will likely be incorporated. The interface module 120 may be configured to instruct the communication module 108 to adjust waveform characteristics of the wave. The interface module 120 may be configured to instruct the communication module 108 to toggle power to the system 100. The interface module 120 may be configured to instruct the communication module 108 to adjust waveform intensity of the wave. Thus the communication module may be operable to configure the wave - e.g. under instruction from the user interface 120 - by adjusting the oscillatory wave generator, in a manner that changes the frequency, amplitude and/or intensity of the wave.

The interface module and communication module may be in wireless communication, or may be connected by a wired connection such as connection 602 shown in FIG. 6.

The system 100 may assemble into a fully intranasal system with minimal external componentry. The design of system 100 is not limited to a fully intranasal system. For example, any or all of the modules such as the power module 106, oscillatory wave generator 102, housing 202, and communication module 108 may be located outside the nasal cavity and, in the nasal passage, are provided components for secondary therapy communication - i.e. components capable of transmitting the therapy that may be, but are not limited to, acoustic or electrical waves. The system 100 may thus enable:
the use of pressure waveforms to loosen mucus in order to achieve mucus mobilisation;
customisable waveforms to allow user control of operating parameters in order to achieve desired treatment outcomes;
hands-free and passive operation which does not require patient active involvement during therapy;
discreet, non-obtrusive or non-obvious form factor; and/or
mess-free use to improve patient convenience and experience.

FIGs. 5a to 5d show various embodiments for, for example, the system 100 of FIG. 1.

FIG. 5a shows an embodiment in which the system 500 comprises a patch 506 for attachment to facial skin of the subject, the patch comprising at least one of the oscillatory wave generator, power module and communication module. In this embodiment, each component - e.g. modules 102, 106, 108, 120 and any other additional functional modules 122, and submodules 124 -is housed in either the patch 500 or intranasal module (which may also be referred to as an intranasal therapy transmitter) 502, or potentially in wave guide 504.

FIG. 5b shows an embodiment in which all components are housed within a face mask 508. The nasal module 520 comprises is adapted to engage or sit on the outside of the nose - i.e. is an extranasal module - and deliver the wave into the nasal passage through the nostril(s). The extranasal module 520 may comprise a U-shaped portion, which may be resilient, that snugly fits over the end or bridge of the nose to maintain the position of the acoustic generator at the nostril(s). The extranasal module 520 may instead be held in place by mask 508. This embodiment may also be used with an intranasal module (not shown) with a wave guide (if provided or necessary) extending from the intranasal module to the mask 508 or components, e.g. wave generator (not shown) in the mask 508.

FIG. 5c shows an embodiment comprising an intranasal module 510, connected with an external housing 512 via a wave guide 514. The housing 512 is shaped to be received behind the ear 516 of the subject.

FIG. 5d shows an embodiment comprising an intranasal module 518. The intranasal module may comprise the wave generator, communication module, power module and other necessary components. Alternatively, the intranasal module 518 may communicate wirelessly, via the communication module, with the wave generator.

FIG. 6 shows an experimental setup of the system for airway secretion management. The user interface 600 is provided in a smartphone 601 connected by a wire 602 to a board 604. The board 604 is connected to a power module, presently comprising a D battery 606. The board 604 or smartphone 601 comprise the communication module and the waveform generator and amplifier. The acoustic generator 608 is attached via a wired connection 610 to the board 604, and forms part of the intranasal module 612. In the present embodiment, the intranasal module 612 comprises a conduit 614 through which waves produced by the acoustic generator 608 are propagated into the subject.

The components of the system of FIG. 6 operate as described above.

The system of FIG. 6 is designed to be worn transiently by the user and removed once the session is completed. The physical embodiment of such a system may be as shown in FIGs. 2 and 3, in which the external housing contains some or all modules of the system and is secured on the body of the user in a hands-free manner while the acoustic oscillations transmitter (i.e. acoustic generator) is fixed in one of the nostrils. The device is a powered electronics system capable of delivering acoustic oscillations in a fully-automated manner, thereby removing the need for human participation by the patient or caregiver. Additionally, unlike existing solutions that limit the user's freedom of motion, this solution allows the user to receive therapy in a highly lifestyle integrative manner, enabling them to continue with their important daily activities in a hands-free and mobility friendly manner. By allowing for therapy "in-the-background" while conducting other activities, it is designed to maintain good respiratory hygiene in an effortless and time-saving manner. It is intended the system may therefore assist with maintaining treatment compliance.

FIG. 7 shows the positioning of an intranasal module 700 in the nasal passage of a subject 702. The system thus enables a (i) less convoluted route for pressure wave delivery and (ii) discrete and readily accessible location of the intranasal module 700. The less convoluted route or pathway into the airways is intended to reduce wave attenuation before the wave arrives at the site of the secretion.

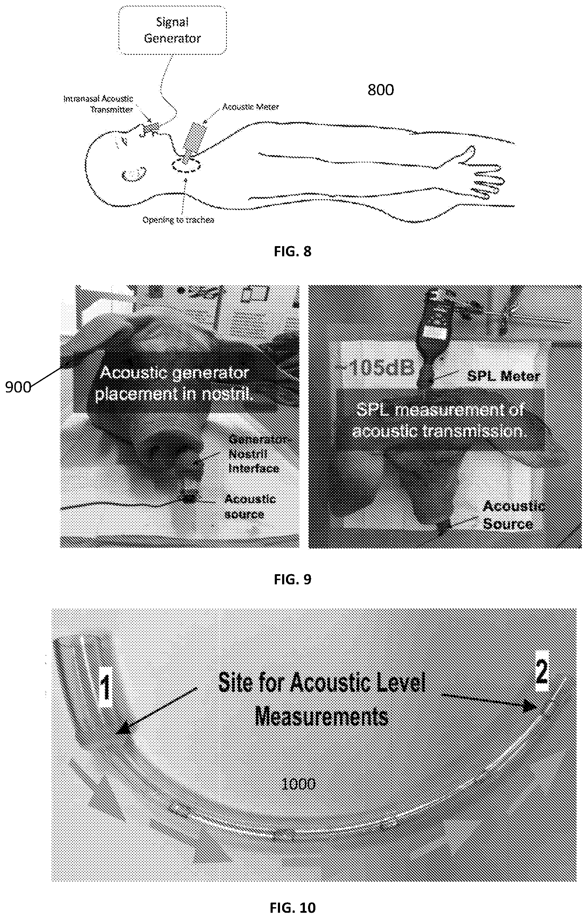

FIGs. 8 to 10 show a human cadaver model, porcine cadaver model and bench model used in experimentally determining efficacy of the experimental model shown in FIG. 6. FIG. 8 demonstrates the experiment design to evaluate acoustic attenuation in the nasopharyngeal region of a human cadaver 800. An intranasal module 802 comprising the acoustic or signal generator 804 was inserted into the nasal passage of the cadaver 800, and an acoustic meter 806 was placed in an opening to the trachea 808 to measure the strength of the wave in the trachea, as propagated from the intranasal module.

The airway was first identified and the absence of any obstruction was confirmed. SPL measurements were performed at 50Hz at the point of source (105.8 dB) and the tracheal opening (93 dB), respectively.

FIG. 9 demonstrates the experiment design to evaluate acoustic attenuation in the nasopharyngeal region of a porcine cadaver 900. The airway was first identified and the absence of any obstruction was confirmed. SPL measurements were performed at 50Hz at the point of source (107dB) and the tracheal opening (105dB), respectively.

FIG. 10 is an image of the experiment design to evaluate acoustic attenuation as a function of distance in a bench model. A conduit 1000 was created to mimic open access from the nostril to trachea in a subject, and being approximately the same length as the distance between those points in the subject. SPL measurements were performed at 50Hz at the point of source (107db) and the tracheal opening (105dB), respectively.

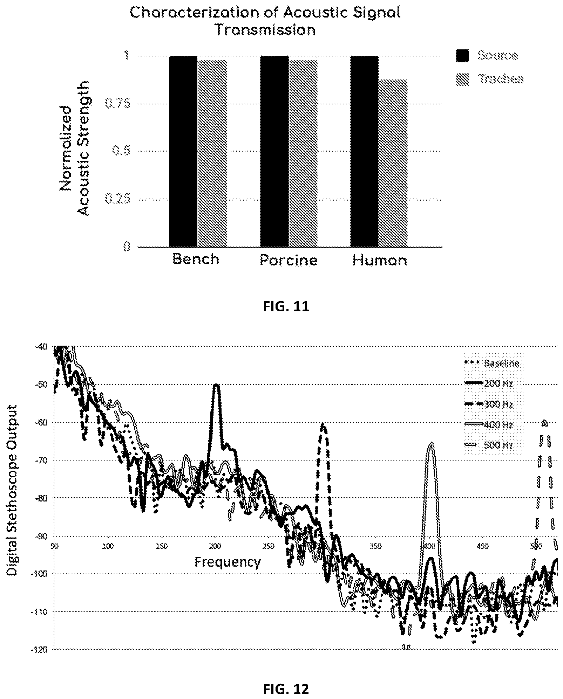

FIG. 11 shows the experiment outcomes obtained from the bench 1000, porcine 900, and human cadaver 800 models. As illustrated, variations of acoustic strength were identified when the acoustic oscillation was transmitted from the source to the point of measurement. Both bench and porcine models demonstrated <2% signal reduction while human cadaver model experienced <15% signal reduction. The signal reduction, particularly in the human cadaver model, can be compensated for by adjusting the power input delivered to the acoustic generator.

FIG. 12 illustrates the acoustic spectra recorded by a digital stethoscope in the region of the anterior apex of the right lung of a subject. As shown, the nasal passage offers a more direct route to the central airways, when compared with the oral cavity. Signals deliver intraorally may thus suffer from greater attenuation, particularly when the mouth is completely shut as shown in FIG. 5a. As a result, intranasal routes may be more effective for acoustic delivery.

The experiment design was for determining transmission of acoustics as a function of frequencies. Here, acoustic waves at 200, 300, 400, and 500 Hz were generated at the source. Acoustics were captured and recorded using a digital stethoscope. The acoustic generator was placed in the nostril. The stethoscope readings were obtained from the anterior apex of the right lung in a supine position.

FIG. 5b shows experimental results for acoustic spectra detected in the apical region of the right lung. This was used to evaluate the transmissibility of acoustic waves through nasal routes. For these experiments, model pressure waveforms were generated at 300 Hz and 500 Hz. Distinct peaks could be observed at 300 and 500 Hz, suggesting fidelity of transmitted signals.

The systems taught herein were developed on the basis that is was found the nasal passage provides ease-of-access and adequate space for the placement of intranasal devices. The nasal passage was then determined to be preferred, or ideal, when compared with the oral cavity for delivery of waves for the purpose of airway secretion management.

The present teachings may be used to produce a device or system for airway secretion management as described herein.

Many modifications will be apparent to those skilled in the art without departing from the scope of the present invention, which is defined by the claims which follow.

Throughout this specification, unless the context requires otherwise, the word "comprise", and variations such as "comprises" and "comprising", will be understood to imply the inclusion of a stated integer or step or group of integers or steps but not the exclusion of any other integer or step or group of integers or steps.

## Claims

1. A system (100) for respiratory system secretion management, comprising:
an oscillatory wave generator (102) for generating a wave for loosening secretion from a respiratory system of a subject; and
a power module (106) configured to power the system;
an intranasal module (104) in communication with the oscillatory wave generator (102), for delivering the wave into a nasal passage of the subject;
a communication module (108) coupled to at least one of the intranasal module (104) and the oscillatory wave generator (102), for selectively communicating power and/or one or more waveform characteristics to the respective intranasal module and/or oscillatory wave generator; and
an interface module (120) in wireless communication with the communication module, for providing instructions to the communication module, thereby to control the communication module,
wherein the intranasal module (104) is shaped to be positioned in the nasal passage and comprises a housing (402) shaped to seal against the nasal passage, and an acoustic window (406) at a distal end of the housing, through which the wave is delivered into the nasal passage;
wherein the intranasal module (104) is in communication with the oscillatory wave generator (102) via a waveguide; and
an external housing (202) connected to the intranasal module (104) by the waveguide, the oscillatory wave generator (102) being housed in the external housing (202), the waveguide being flexible to permit flexible placement of the housing (202) during delivery of therapy.

2. A system according to claim 1, wherein the oscillatory wave generator comprises a waveform generator (110) for generating an electrical signal corresponding to the wave, and at least one of:
an amplifier (112) for increasing an amplitude and/or power of the electrical signal; and
an acoustic generator (114) for converting the electrical signal into an acoustic signal, said acoustic signal being the wave.

3. A system according to claim 1, wherein the oscillatory wave generator (102) is adjustable to control output waveform characteristics.

4. A system according to claim 3, wherein the waveform characteristics comprises one or more of frequency, amplitude, intensity, pressure, duration of use.

5. A system according to claim 1, wherein the intranasal module (104) is adapted to grip the nasal passage.

6. A system according to claim 1, wherein the intranasal module comprises an anchor component (410) for preventing irretrievable slippage of the intranasal module into the nasal passage

7. A system according to claim 6, wherein the anchor component (410) comprises a hook for catching onto a columella of the subject.

8. A system according to claim 1, wherein the interface module (120) is configured to instruct the communication module (108) to at least one of:
adjust waveform characteristics of the wave; and
toggle power to the system.

9. A system according to claim 1, comprising a patch (506) for attachment to facial skin of the subject, the patch comprising at least one of the oscillatory wave generator, power module and communication module.

10. The system of any one of claims 1 to 9, wherein the oscillatory wave generator generates a square wave.

## Patentansprüche

1. System (100)
zum Verwalten von Atemwegssystemsekretion, umfassend:
einen Oszillationswellengenerator (102) zum Erzeugen einer Welle zum Lösen von Sekret aus dem Atemwegssystem eines Subjekts; und
ein Leistungsmodul (106), das zur Stromversorgung des Systems konfiguriert ist;
ein intranasales Modul (104), das mit dem Oszillationswellengenerator (102) in Verbindung steht, um die Welle in einen Nasengang des Subjekts zuzuführen;
ein Kommunikationsmodul (108), das mit mindestens einem aus dem intranasalen Modul (104) und dem Oszillationswellengenerator (102) gekoppelt ist, um selektiv Leistung und/oder eine oder mehrere Wellenformcharakteristiken an das jeweilige intranasale Modul und/oder den Oszillationswellengenerator zu übermitteln; und
ein Schnittstellenmodul (120), das in drahtloser Verbindung mit dem Kommunikationsmodul steht, um Anweisungen an das Kommunikationsmodul bereitzustellen und dadurch das Kommunikationsmodul zu steuern, wobei das intranasale Modul (104) geformt ist, um in dem Nasenkanal positioniert zu werden, und ein Gehäuse (402), das geformt ist, um an dem Nasenkanal abzudichten, und ein akustisches Fenster (406) an einem distalen Ende des Gehäuses, durch das die Welle in den Nasenkanal zugeführt wird, umfasst;
wobei das intranasale Modul (104) über einen Wellenleiter mit dem Oszillationswellengenerator (102) in Verbindung steht; und
ein externes Gehäuse (202), das durch den Wellenleiter mit dem intranasalen Modul (104) verbunden ist, wobei der Oszillationswellengenerator (102) in dem externen Gehäuse (202) untergebracht ist, wobei der Wellenleiter flexibel ist, um eine flexible Platzierung des Gehäuses (202) während eines Zuführens von Therapie zu ermöglichen.

2. System nach Anspruch 1, wobei der Oszillationswellengenerator einen Wellenformgenerator (110) zum Erzeugen eines der Welle entsprechenden elektrischen Signals und mindestens eines von Folgenden umfasst:
einen Verstärker (112) zum Erhöhen einer Amplitude und/oder Leistung des elektrischen Signals; und
einen akustischen Generator (114) zum Umwandeln des elektrischen Signals in ein akustisches Signal, wobei das akustische Signal die Welle ist.

3. System nach Anspruch 1, wobei der Oszillationswellengenerator (102) anpassungsfähig ist, um Eigenschaften der Ausgangswellenform zu steuern.

4. System nach Anspruch 3, wobei die Wellenformeigenschaften eines oder mehrere von Frequenz, Amplitude, Intensität, Druck, Dauer der Anwendung umfassen.

5. System nach Anspruch 1, wobei das intranasale Modul (104) angepasst ist, um den Nasenkanal zu greifen.

6. System nach Anspruch 1, wobei das intranasale Modul eine Verankerungskomponente (410) umfasst, um ein unwiederbringliches Verrutschen des intranasalen Moduls in den Nasenkanal zu verhindern.

7. System nach Anspruch 6, wobei die Verankerungskomponente (410) einen Haken zum Einhaken in eine Columella des Subjekts umfasst.

8. System nach Anspruch 1, wobei das Schnittstellenmodul (120) konfiguriert ist, um das Kommunikationsmodul (108) zu mindestens einem von Folgenden anzuweisen:
Anpassen der Wellenformeigenschaften der Welle; und
Zuschalten von Strom zu dem System.

9. System nach Anspruch 1, umfassend ein Pflaster (506) zum Anbringen auf der Gesichtshaut des Subjekts, das Pflaster umfassend mindestens eines von dem Oszillationswellengenerator, Energiemodul und Kommunikationsmodul.

10. System nach einem der Ansprüche 1 bis 9, wobei der Oszillationswellengenerator eine Rechteckwelle erzeugt.

## Revendications

1. Système (100) de gestion des sécrétions du système respiratoire, comprenant :
un générateur d'ondes oscillatoires (102) pour générer une onde pour relâcher la sécrétion d'un système respiratoire d'un sujet ; et
un module d'alimentation (106) configuré pour alimenter le système ;
un module intranasal (104) en communication avec le générateur d'ondes oscillatoires (102), pour délivrer l'onde dans un passage nasal du sujet ;
un module de communication (108) couplé à au moins l'un du module intranasal (104) et du générateur d'ondes oscillatoires (102), pour communiquer sélectivement l'alimentation et/ou une ou plusieurs caractéristiques de forme d'onde au module intranasal et/ou au générateur d'ondes oscillatoires respectif ; et
un module d'interface (120) en communication sans fil avec le module de communication, pour fournir des instructions au module de communication, afin de commander ainsi le module de communication,
dans lequel le module intranasal (104) est profilé pour être positionné dans le passage nasal et comprend un boîtier (402) profilé pour assurer l'étanchéité contre le passage nasal, et une fenêtre acoustique (406) au niveau d'une extrémité distale du boîtier, à travers laquelle l'onde est délivrée dans le passage nasal ;
dans lequel le module intranasal (104) est en communication avec le générateur d'ondes oscillatoires (102) via un guide d'ondes ; et
un boîtier externe (202) relié au module intranasal (104) par le guide d'ondes, le générateur d'ondes oscillatoires (102) étant logé dans le boîtier externe (202), le guide d'ondes étant flexible pour permettre un placement flexible du boîtier (202) pendant l'administration de la thérapie.

2. Système selon la revendication 1, dans lequel le générateur d'ondes oscillatoires comprend un générateur de forme d'onde (110) pour générer un signal électrique correspondant à l'onde, et au moins l'un parmi :
un amplificateur (112) pour augmenter l'amplitude et/ou la puissance du signal électrique ; et
un générateur acoustique (114) pour convertir le signal électrique en un signal acoustique, ledit signal acoustique étant l'onde.

3. Système selon la revendication 1, dans lequel le générateur d'ondes oscillatoires (102) est réglable pour commander les caractéristiques de forme d'onde de sortie.

4. Système selon la revendication 3, dans lequel les caractéristiques de forme d'onde comprennent une ou plusieurs parmi la fréquence, l'amplitude, l'intensité, la pression et la durée d'utilisation.

5. Système selon la revendication 1, dans lequel le module intranasal (104) est adapté pour agripper le passage nasal.

6. Système selon la revendication 1, dans lequel le module intranasal comprend un composant d'ancrage (410) pour empêcher un glissement irréversible du module intranasal dans le passage nasal.

7. Système selon la revendication 6, dans lequel le composant d'ancrage (410) comprend un crochet pour s'accrocher à une columelle du sujet.

8. Système selon la revendication 1, dans lequel le module d'interface (120) est configuré pour donner au module de communication au moins une instruction parmi :
ajuster des caractéristiques de forme d'onde de l'onde ; et
basculer l'alimentation au système.

9. Système selon la revendication 1, comprenant un patch (506) pour fixation sur la peau du visage du sujet, le patch comprenant au moins l'un parmi le générateur d'ondes oscillatoires, le module d'alimentation et le module de communication.

10. Système selon l'une quelconque des revendications 1 à 9, dans lequel le générateur d'ondes oscillatoires génère une onde carrée.
